Europäisches Patentamt

European Patent Office

Office européen des brevets

(19)

(11) Numéro de publication : **0 512 040 B1**

(12) **FASCICULE DE BREVET EUROPEEN**

(45) Date de publication du fascicule du brevet :
**03.11.93 Bulletin 93/44**

(51) Int. Cl.⁵ : **A61K 7/48**

(21) Numéro de dépôt : **91903709.3**

(22) Date de dépôt : **22.01.91**

(86) Numéro de dépôt international :
**PCT/FR91/00036**

(87) Numéro de publication internationale :
**WO 91/11169 08.08.91 Gazette 91/18**

(54) **PREPARATIONS COSMETIQUES CONTENANT UN EXTRAIT DE TOURTEAUX DE TOURNESOL (HELIANTHUS ANNUUS).**

(30) Priorité : **24.01.90 FR 9001024**

(43) Date de publication de la demande :
**11.11.92 Bulletin 92/46**

(45) Mention de la délivrance du brevet :
**03.11.93 Bulletin 93/44**

(84) Etats contractants désignés :
**AT BE CH DE DK ES FR GB GR IT LI LU NL SE**

(56) Documents cités :
**FR-A- 2 207 652
FR-A- 2 487 674
GB-A- 2 067 899
PATENT ABSTRACTS OF JAPAN, vol. 13, N:o 63 (C-568)(3411), 13 February 1989, & JP-A-63255212
S.T.N., Serveur de Bases de Données, Fichies Chemical Abstracts, vol. 109, 10 October 1988, Karlsruhe, DE, see abstract 127506u, M. SAEED et al.: "Sunflower protein concentrates and isolates low in polyphenols and phytate"**

(56) Documents cités :
**Parfums, Cosmétiques, Arômes, N:o 56, April-May 1984, J. RAYMOND et al.: Les protéines d'oléagineaux: Prpriétés fonctionelles utilisables en cosmétologie", page 57, see the abstract cited in the application
S.T.N. Serveur de Bases de Données, Fichies Chemical Abstracts, vol. 98, 3 January 1983, Karlsruhe, DE, see abstract 3743d, G. SRIPAD et al.: "Extractability of polyphenols of sunflower seed in various solvents"**

(73) Titulaire : **Bonne, Claude
316, avenue d'Occitanie
F-34000 Montpellier (FR)**
Titulaire : **Sincholle, Daniel
343, avenue de la Trémoulette
F-34980 Saint-Clement (FR)**
Titulaire : **Diot, Michel
2, allée des Dimanches
F-78430 Louveciennes (FR)**

(72) Inventeur : **Bonne, Claude
316, avenue d'Occitanie
F-34000 Montpellier (FR)**
Inventeur : **Sincholle, Daniel
343, avenue de la Trémoulette
F-34980 Saint-Clement (FR)**
Inventeur : **Diot, Michel
2, allée des Dimanches
F-78430 Louveciennes (FR)**

(74) Mandataire : **Le Guen, Gérard et al
CABINET LAVOIX 2, place d'Estienne d'Orves
F-75441 Paris Cédex 09 (FR)**

## Description

La présente invention concerne une composition cosmétique s'opposant aux effets néfastes de l'exposition au soleil et principalement au vieillissement actinique de la peau.

La présente invention concerne plus particulièrement une composition cosmétique contenant à titre de principe actif un extrait de tourteaux de tournesol (Helianthus annuus).

Le tournesol, Helianthus annuus, de la famille des Astéracées, est une plante d'une hauteur variant de 1 mètre à 1,50 mètre, très connue en Europe pour ses graines, qui fournissent, lorsqu'elles sont cueillies à maturité, une huile alimentaire (huile de tournesol) et un résidu que l'on appelle tourteau, utilisé en alimentation animale.

Certains ont trouvé des propriétés, intéressantes en cosmétologie, à un isolat protéinique provenant de tourteaux de tournesol (Raymond et coll. - Parfums, Cosmétiques, Arômes - n° 56, avril-mai 1984, p. 57), mais n'envisagent pas l'utilisation d'une fraction riche en polyphénols telle que celle utilisée dans l'invention.

De plus, ces mêmes auteurs ont trouvé à ces protéines des propriétés émulsifiantes, mais n'ont pas trouvé des propriétés biologiques nouvelles à la fraction provenant de tourteaux de tournesol utilisée dans la présente invention.

D'autres encore (Nedeczky et coll., FR-A-2 487 674) pensent pouvoir utiliser les extraits de tiges de tournesol, et non pas les graines, comme matière première en cosmétologie.

Il est évident, à la vue des résultats présentés plus loin, que l'extrait de tiges, réalisé dans les mêmes conditions que l'extrait de tourteaux de tournesol, n'a pas les mêmes propriétés biologiques.

Certains auteurs (Malvoisin, FR-A-2 207 652) ont aussi pensé pouvoir utiliser comme matière première de l'industrie cosmétique des graines utilisables dans l'alimentation, ce qui permettrait d'obtenir des liquide de base troubles.

L'extrait utilisé dans la présente invention est caractérisé notamment par sa limpidité, parce qu'il ne s'agit absolument pas d'une émulsion et, contrairement à d'autres extraits, l'extrait utilisé est obtenu à partir d'une matière première provenant d'une graine riche en matière grasse dont on a précisément retiré les matières grasses, précipité les protéines et extrait les substances restantes par un alcool tel que le méthanol, de façon à obtenir un extrait riche en polyphénols.

Enfin, un brevet d'invention (L'Oréal, GB-A-2 067 899) mentionne la possibilité d'utiliser, comme matière première pour la cosmétologie, ce qu'il reste de plante médicinale après extraction de différents principes : huiles essentielles, colorants, principes actifs. Il faut cependant remarquer que ce brevet prévoit l'utilisation du résidu sans aucune préparation.

Or, précisément, l'extrait utilisé dans la présente invention est obtenu à la suite de deux extractions successives. La première extraction consiste à retirer l'huile de la graine de tournesol et la deuxième extraction consiste à extraire les résidus provenant de la première extraction.

L'extrait utilisé dans la présente invention est un extrait alcoolique de tourteaux de tournesol. Il peut être obtenu à partir de tourteaux de tournesol, dont on a extrait l'huile, par pulvérisation des tourteaux et extraction par un alcool, notamment le méthanol.

L'extraction peut être réalisée à température ambiante par agitation prolongée (par exemple 24 heures), en utilisant un rapport tourteaux/alcool d'environ 1 partie en poids de tourteaux pour 10 à 50 parties en volume d'alcool.

L'exemple suivant illustre la préparation de l'extrait.

### Exemple de préparation

On broye des tourteaux de tournesol obtenus à la suite d'une opération d'extraction de l'huile, jusqu'à l'obtention d'une taille telle que le refus au tamis de 500 micromètres soit inférieur à 5 %. On ajoute à 100 g de la poudre ainsi obtenue 2 litres de méthanol et on agite l'ensemble pendant 24 heures à température ambiante. On filtre et on évapore à sec le filtrat. On obtient 10 g de résidu que l'on reprend dans 30 g de 1,2-propanediol. On obtient un extrait ayant une teneur de polyphénols de 2,5 % en poids.

Les Demandeurs ont découvert que l'extrait de tourteaux de tournseol utilisé dans la présente invention possède une activité inhibitrice des réactions photochimiques (peroxydations) et, qu'en conséquence, des compositions cosmétiques contenant cet extrait à une concentration en polyphénols de 0,01 à 1 % en poids, de préférence de 0,1 à 0,5 % en poids peuvent s'opposer aux effets néfastes de l'exposition solaire et particulièrement au vieillissement actinique de la peau.

Lorsque la peau est exposée à la lumière solaire, les ultraviolets, mais également les radiations du spectre visible, sont capables d'induire dans ses différentes couches des phénomènes photochimiques conduisant par réactions radicalaires en chaîne à des altérations tissulaires comparables à celles que produit le vieillissement.

Il est reconnu que l'exposition solaire chronique accélère le vieillissement cutané. La prévention de ce vieillissement actinique n'est qu'en partie résolue par l'application de filtres ultraviolets, ceux-ci n'ayant qu'un spectre d'absorption limité. Les radiations visibles absorbées par des molécules endogènes telles que les dérivés flaviniques sont également susceptibles d'induire des réactions photochimiques qui contribuent à l'action nocive d'une exposition solaire excessive.

Le vieillissement d'un tissu peut être objectivé par l'accumulation, en son sein, de produits de peroxydation lipidique appelés lipofuscine ou "pigment de vieillesse" résultat de réactions radicalaires induites par des phénomènes enzymatiques (oxydasiques) ou non-enzymatiques (photochimiques).

Les Demandeurs ont mis en évidence que l'extrait utilisé dans l'invention s'opposait au processus d'oxydation lipidique.

La présente invention a par conséquent pour objet une composition cosmétique s'opposant au vieillissement de la peau, caractérisée en ce qu'elle contient un extrait alcoolique de tourteaux de tournesol, à une concentration exprimée en polyphénols de 0,01 à 1 % en poids, dans une base cosmétique appropriée.

Les expériences rapportées ci-après mettent en évidence l'activité de l'extrait, principe actif utilisé dans la présente invention.

Peroxydation épidermique photo-induite :

Les pigments lipofusciniques qui s'accumulent dans les tissus senescents sont des complexes lipoprotéiques formés par des produits de peroxydation lipidique liés aux résidus aminés des protéines membranaires. Lorsqu'une peroxydation aigue est induite dans un tissu, l'on peut mettre en évidence peu après une augmentation de produits de peroxydation grâce à leur propriété de former, avec l'acide thiobarbiturique, un composé absorbant à 532 nm. En se fondant sur ces principes, les demandeurs ont élaboré un protocole expérimental démontrant l'activité antiperoxydante de l'extrait de tourteaux de tournesol.

Les tests ont été réalisés chez la souris atrichis, photosensibilisée par le rose bengale. Après immersion dans une solution de rose bengale (0,01 M) pendant 5 minutes, les animaux ont été séchés à l'obscurité pendant 10 minutes, puis une plage de peau dorsale a été traitée par application de 25 μl de préparation. Une autre plage servant de contrôle n'a reçu que l'excipient. Les animaux ont été ensuite exposés pendant 30 minutes à la lumière d'une lampe Kripton de 75 watts placée à 25 cm.

Les souris ont été tuées par dislocation cervicale et des échantillons de peau (traitée et témoin) ont été prélevés. L'épiderme a été collectée après décollement à 55° C pendant 30 secondes. Les échantillons ont été homogénéisés dans un tampon, déféqués par l'acide trichloracétique et centrifugés.

Un échantillon d'homogénat a été conservé pour le dosage des protéines. Dans le surnageant obtenu, un volume égal d'acide thiobarbiturique a été ajouté pour que sa concentration finale soit de 0,35 %. Après chauffage à 100° C pendant 15 minutes, la coloration développée a été mesurée au spectrophotomètre, à 532 nm. L'effet protecteur de l'extrait vis-à-vis du processus peroxydatif est illustré dans le Tableau I.

Les produits testés sont :
- l'excipient constitué par du 1,2-propanediol,
- l'excipient contenant 10 % d'un extrait de tourteaux de tournesol contenant 2,5 % en poids de polyphénols,
- l'excipient contenant 2 % du même extrait de tourteaux,
- l'excipient contenant 10 % d'extrait de tiges de tournesol (obtenu dans les mêmes conditions que dans l'exemple de préparation ci-dessus).

## TABLEAU I

### Effet protecteur de l'extrait de tourteaux de tournesol vis-à-vis de la peroxydation épidermique photo-induite

| Traitement | Réactifs de l'acide thiobarbiturique (DO/10 mg protéine $ml^{-1}$) | Inhibition (%) |
|---|---|---|
| Excipient | $0,105 \pm 0,010$ | – |
| Extrait de tourteaux 10 % | 0 | 100 |
| Extrait de tourteaux 2 % | $0,045 \pm 0,010$ | 52 |
| Extrait de tiges 10 % | $0,003 \pm 0,001$ | 3 |

Les compositions cosmétiques selon l'inven- tion peuvent contenir éventuellement toutes les substances connues pour leurs propriétés bienfaisantes en cosmétologie et particulièrement des filtres solaires, des hydratants, des acides hyaluroniques, des lipides, phospholipides, des vitamines, des parfums, des conservateurs et des colorants.

Les compositions cosmétiques selon l'invention peuvent se présenter sous toutes les formes utilisées en cosmétologie : crème, gel, lait, lotion en pot, en tube, en ampoule, en nébuliseur et en bâton.

Les différentes formes cosmétiques mentionnées ci-dessus sont obtenues selon les méthodes utilisées dans ce domaine.

Ci-après sont rapportés des exemples de compositions cosmétiques (en parties en poids) :

1) Crème H/E

| | |
|---|---|
| Extrait contenant 2,5 % de polyphénols | 0,5 à 10 |
| Huile de germe de blé | 8 |
| Stéarate de glycérol | 4 |
| Triéthanolamine | 0,5 |
| Polymère carboxyvinylique | 0,5 |
| Esters méthoxycinnamiques | qs |
| Benzophénone | qs |
| Conservateurs | qs |
| Eau | qsp 100 |

2) <u>Gel</u>

```
Extrait contenant 2,5 % de polyphénols    0,5 à 10
Glycérol                                   40
Alginate de sodium                         7
Borax                                      1
Thymol                                     qs
Conservateurs                              qs
Eau                                        qsp 100.
```

**Revendications**

1. Composition cosmétique s'opposant au vieillissement de la peau, caractérisée en ce qu'elle contient un extrait alcoolique de tourteaux de tournesol, à une concentration exprimée en polyphénols de 0,01 à 1 % en poids, dans une base cosmétique appropriée.

2. Composition selon la revendication 1, caractérisée en ce que l'extrait est un extrait par le méthanol.

3. Composition selon la revendication 1 ou la revendication 2, contenant ledit extrait à une concentration exprimée en polyphénols de 0,1 à 0,5 % en poids.

4. Utilisation d'un extrait alcoolique de tourteaux de tournesol pour la fabrication d'une composition cosmétique s'opposant au vieillissement de la peau selon la revendication 1.

5. Procédé pour s'opposer au vieillissement de la peau, dans lequel on applique sur la peau une composition cosmétique contenant un extrait alcoolique de tourteaux de tournesol à une concentration exprimée en polyphénols de 0,01 à 1 % en poids, dans une base cosmétique appropriée.

6. Procédé selon la revendication 5, dans lequel l'extrait est un extrait par le méthanol.

7. Procédé de préparation d'une composition cosmétique selon la revendication 1, dans lequel on incorpore un extrait alcoolique de tourteaux de tournesol à une concentration exprimée en polyphénols de 0,01 à 1 % en poids dans une base cosmétique appropriée.

8. Procédé selon la revendication 7, dans lequel l'extrait est un extrait par le méthanol.

**Claims**

1. Cosmetic composition counteracting ageing of the skin, characterized in that it contains an alcoholic extract of sunflower oil cake at a concentration expressed as 0.01 to 1% by weight of polyphenols, in a suitable cosmetic base.

2. Composition according to Claim 1, characterized in that the extract is a methanol extract.

3. Composition according to Claim 1 or Claim 2, containing the said extract at a concentration expressed as 0.1 to 0.5% by weight of polyphenols.

4. Use of an alcoholic extract of sunflower oil cake for the manufacture of a cosmetic composition counteracting ageing of the skin according to Claim 1.

5. Process for counteracting ageing of the skin, wherein a cosmetic composition containing an alcoholic extract of sunflower oil cake at a concentration expressed as 0.01 to 1% by weight of polyphenols, in a suitable cosmetic base, is applied to the skin.

6. Process according to Claim 5 wherein the extract is a methanol extract.

7. Process for the preparation of a cosmetic composition according to Claim 1, wherein an alcoholic extract of sunflower oil cake is incorporated into a suitable cosmetic base at a concentration expressed as 0.01 to 1% by weight of polyphenols.

8. Process according to Claim 7 wherein the extract is a methanol extract.

**Patentansprüche**

1. Kosmetikzusammensetzung zur Verhinderung der Hautalterung, dadurch charakterisiert, daß sie einen alkoholischen Extrakt der Ölkuchens der Sonnenblume in einer Konzentration, ausgedrückt in Polyphenolen, von 0.01 bis 1 Gew.% in einer geeigneten Kosmetikbasis enthält.

2. Zusammensetzung nach Anspruch 1. dadurch charakterisiert, daß der Extrakt ein Methanol-Extrakt ist.

3. Zusammensetzung nach Anspruch 1 oder Anspruch 2, enthaltend den besagten Extrakt in einer Konzentration von 0.1 bis 0.5 Gew.%, ausgedrückt in Polyphenolen.

4. Verwendung eines alkoholischen Extrakts des Ölkuchens der Sonnenblume für die Herstellung einer Kosmetikzusammensetzung zur Verhinderung der Hautalterung nach Anspruch 1.

5. Verfahren zur Verhinderung der Hautalterung, bei dem auf die Haut eine Zusammensetzung aufgetragen wird, enthaltend einen alkoholischen Extrakt des Ölkuchens Sonnenblume in einer geeigneten Kosmetikbasis in einer Konzentration, ausgedrückt in Polyphenolen, von 0.01 bis 1 Gew. %.

6. Verfahren nach Anspruch 5, in dem der Extrakt ein Methanol-Extrakt ist.

7. Verfahren zur Herstellung einer Zusammensetzung nach Anspruch 1, in dem ein alkoholischer Extrakt des Ölkuchens der Sonnenblume in einer Konzentration, ausgedrückt in Polyphenolen, von 0.01 bis 1 Gew.% in eine geeignete Kosmetikbasis eingebracht wird.

8. Verfahren nach Anspruch 7, in dem der Extrakt ein Melhanol-Extrakt ist.